# Europäisches Patentamt
# European Patent Office
# Office européen des brevets

(19)

(11) Veröffentlichungsnummer: **0 205 870**
**B1**

(12) ## EUROPÄISCHE PATENTSCHRIFT

(45) Veröffentlichungstag der Patentschrift:
19.04.89

(51) Int. Cl.⁴: **B 60 T 17/22**, G 01 N 33/28

(21) Anmeldenummer: 86106422.8

(22) Anmeldetag: 12.05.86

(54) Einrichtung zur Überwachung von in Hydraulikflüssigkeit eines Bremssystems gelöstem Wasser.

(30) Priorität: 11.06.85 DE 3520858

(43) Veröffentlichungstag der Anmeldung:
30.12.86 Patentblatt 86/52

(45) Bekanntmachung des Hinweises auf die Patenterteilung:
19.04.89 Patentblatt 89/16

(84) Benannte Vertragsstaaten:
DE FR GB IT

(56) Entgegenhaltungen:
DE-A-3 221 403
GB-A-2 139 763

(73) Patentinhaber: **Dr.Ing.h.c. F. Porsche Aktiengesellschaft, Porschestrasse 42, D-7000 Stuttgart 40 (DE)**

(72) Erfinder: **Aydt, Günter, Dipl.- Ing., Sechselberger Weg 3, D-7150 Backnang (DE)**
Erfinder: **Martin, Roland, Dipl.- Ing., Bahnhofstrasse 33, D-7251 Weissach (DE)**

## Beschreibung

Die Erfindung bezieht sich auf eine Einrichtung nach dem Oberbegriff des Anspruchs 1.

Eine ständige Überwachung der Bremsflüssigkeit in einem hydraulischen Druckkreis ist erforderlich, damit der Wasseranteil in der Flüssigkeit feststellbar ist. Dies ist zur Gewährleistung einer gleichbleibenden Funktion der Bremsen unbedingt notwendig. Bei einer Wasseraufnahme in die hygroskopische Bremsflüssigkeit wird der hohe Siedepunkt schon nach wenigen Tagen herabgesetzt und die Bremsflüssigkeit neigt dann zu Dampfblasenbildung, so daß die Bremsflüssigkeit dann nicht mehr ausreichend inkompressibel ist.

Zur Feststellung des Wasseranteils in der Bremsflüssigkeit sind handhabbare Geräte bekannt geworden (DE-A-3 221 403), in denen die Bremsflüssigkeit abgefüllt und eine Untersuchung auf den Wassergehalt vorgenommen werden kann. Diese Geräte werden außerhalb des Bremssystems angewendet und sind nicht Bestandteil dieses Systems. Auch ist mit einem solchen Gerät keine ständige Überprüfung der Bremsflüssigkeit im Kraftfahrzeug möglich.

Desweiteren ist aus der GB-A-2 139 763 eine Vorrichtung zur Überwachung der Beschaffenheit von Druckflüssigkeit bekannt, die in eine druckführende Leitung eingesetzt wird.

Aufgabe der Erfindung ist es, eine Einrichtung zur Überwachung von Bremsflüssigkeit zu schaffen, die eine Überprüfung dieser Bremsflüssigkeit im Kraftfahrzeug und somit auch während des Fahrens ermöglicht.

Diese Aufgabe wird erfindungsgemäß durch die kennzeichnenden Merkmale des Anspruchs 1 gelöst. Weitere vorteilhafte Ausbildungen beinhalten die Unteransprüche.

Die mit der Erfindung hauptsächlich erzielten Vorteile bestehen darin, daß die Überwachungseinrichtung ein ständig im Bremskreis installiertes System ist und der Benutzer eine dauernde Information bekommt, ob die Bremsflüssigkeit noch inkompressibel ist und eine optimale Bremsung gewährleistet wird. Dieses System ist in die Bremsanlage integriert, d.h. in jedem Bremssattel des Systems ist ein Sensor unmittelbar im Druckraum des Bremskolbens vorgesehen. Dies hat den Vorteil einer ständigen Überwachung der Bremsflüssigkeit auf Wasserbestandteile. Damit diese Überwachung auch dem Fahrer unmittelbar übermittelt werden kann, ist eine entsprechende Auswerteeinheit mit Warngerät und mit Signalaufbereitungsschaltung vorgesehen. Eine solche spezielle Anordnung der Sensoren im Druckraum ist von besonderer Bedeutung für die Wirksamkeit der Bremse, da in diesem Druckraum der Bremskolben angeordnet ist und dieser Kolben unmittelbar den Druck steuert. Wenn in diesem Druckraum ein bestimmter Wassergehalt vorhanden ist, kann keine wirksame Bremsung mehr durchgeführt werden. Eine Anordnung der Sensoren irgendwo im System wurde nicht den tatsächlichen Zustand der Bremsflüssigkeit im Wirkbereich der Bremse ergeben.

Die Anzeige über den Zustand der Bremsflüssigkeit kann über ein Anzeigegerät, welches z. B. im Armaturenbrett angeordnet ist, erfolgen. Das Anzeigegerät kann optische oder auch akustische Signale abgeben.

Eine solche ständige Anzeige ist unbedingt erforderlich, da die Versuche gezeigt haben, daß z. B. bei 240 Stunden und einer relativen Feuchte von 60 % die Wasseraufnahme etwa 4,5 Gewichts-% beträgt und gleichzeitig die Siedetemperatur von 300° C auf 130° C absinkt.

Ein Ausführungsbeispiel der Erfindung ist in der Zeichnung dargestellt und wird im folgenden näher beschrieben.

Es zeigen

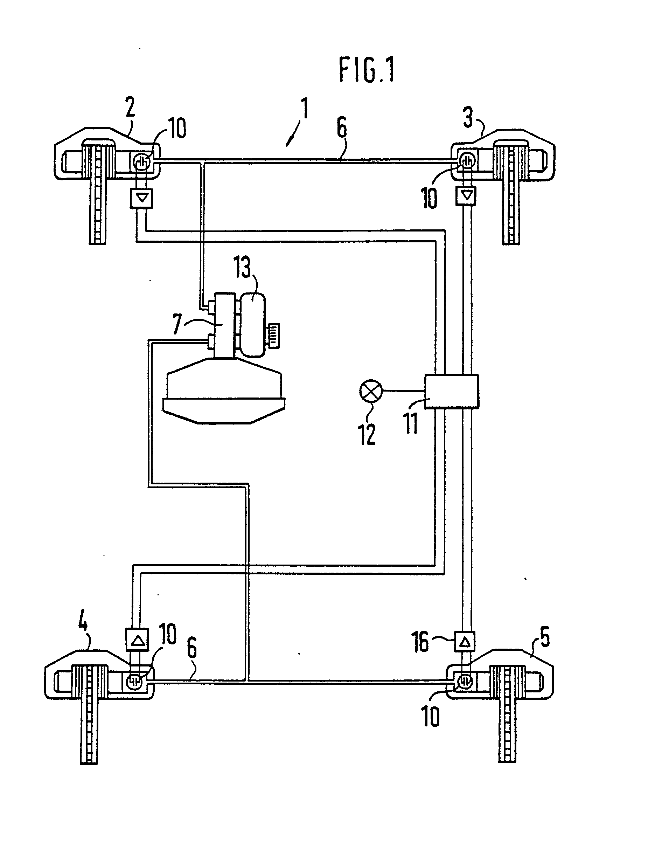

Fig. 1 ein Schema eines Bremssystems für ein Kraftfahrzeug mit einer Sensorschaltung und

Fig. 2 und 3 Draufsichten einer Bremse sowie mit dem Druckraum verbundene Sensoren.

Das Bremssystem 1 eines Kraftfahrzeugs umfaßt im wesentlichen an jeder Fahrzeugachse Bremsen 2, 3, 4 und 5, die mittels eines hydraulischen Kreislaufs über Leitungen 6 mit einem Hauptbremszylinder 7 verbunden sind. Diese Leitungen 6 münden jeweils in Druckräume 8 der jeweiligen Bremse 2, 3, 4 und 5 ein, in denen die Bremskolben 9 angeordnet sind.

Gleichzeitig stehen mit diesen Druckräumen 8 der Bremsen 2 bis 5 Sensoren 10 in Verbindung, denen eine elektronische Auswerteeinheit 11 nachgeschaltet ist. Über ein mit dieser Einheit 11 zusammengeschaltetes Warnanzeigegerät 12 wird ein entsprechendes Signal für den Fahrzeugbenutzer erzeugt. Der Sensor 10 ist z. B. als Plattenkondensator ausgeführt, der seine Signale unter Zwischenschaltung einer Signalaufbereitungsschaltung 16 an die Auswerteeinheit 11 abgibt, die diese Signale entsprechend zur Abgabe eines Warnimpulses an das Gerät 12 weitergibt. Die Signalaufbereitungsschaltung 16 ist vorzugsweise nahe des Sensors 10 angeordnet.

In weiterer Ausgestaltung der Einrichtung kann auch nur ein einziger Sensor im Bremssystem 1 bzw. in dem Druckraum/Leitung 6 bzw. in einem unmittelbar der Bremse zugeordneten Druckraum 8 der Bremse angeordnet sein. Denkbar wäre es ebenfalls, wenn ein Sensor 10 im Nachlaufbehälter 13 vorgesehen ist.

Die Sensoren 10 bzw. die Einheit aus mehreren Sensoren 10 mit der Auswerteeinheit 11 und dem Anzeigegerät 12 sind in jedem Fall fest in das Fahrzeug installiert und in ein Gesamtwarnsystem des Fahrzeugs integriert.

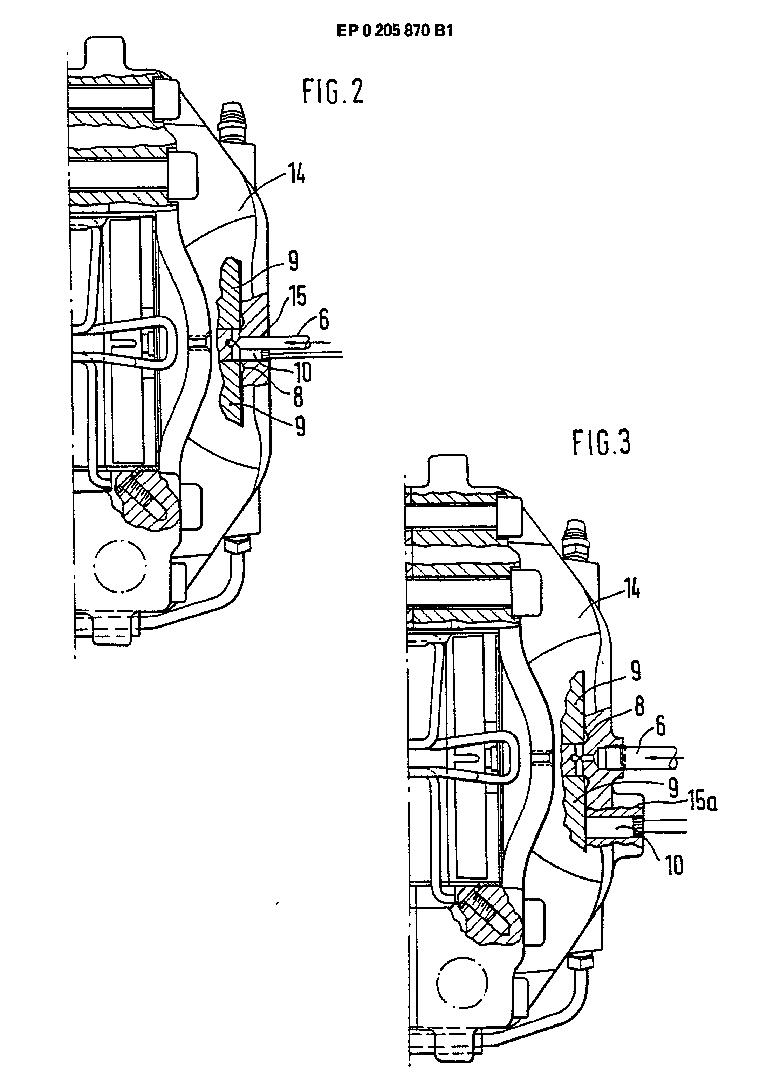

In den Fig. 2 und 3 sind mögliche Anordnungen der Sensoren 10 in einer Bremse im Bremssattel 14 vorgesehen. In Fig. 2 ist der Sensor 10 in der Öffnung 15 gemeinsam mit der Druckleitung 16 angeordnet. In einer weiteren Ausführung gemäß Fig. 3 ist der Sensor 10 separat nahe der Druckleitung 6 in einem Gußansatz 15 des Bremssattels 14 vorgesehen. In jedem Fall ragt der Sensor 10 mit seiner Meßfläche stirnseitig in den Druckraum 8 des Bremskolbens 9.

## Patentansprüche

1. Einrichtung zur Überwachung einer Hydraulikflüssigkeit, insbesondere einer Bremsflüssigkeit in einem Bremssystem eines Fahrzeugs auf enthaltenes Wasser, die in wenigstens einem der Druckräume des Bremssystems angeordnet sind, dadurch gekennzeichnet, daß jeweils ein Sensor (10) im Bremssattel (14) jeder Bremse der Vorder- und Hinterachse des Fahrzeugs angeordnet ist, mit seiner Meßfläche stirnseitig in den Druckraum (8) des Bremskolbens (9) ragt und daß die Sensoren (10) mit einer elektronischen Auswerteeinheit (11) mit Warnanzeigegerät (12) verbunden sind und daß zwischen jedem Sensor (10) und der Auswerteeinheit (11) nahe der Sensoren (10) eine Signalaufbereitungsschaltung (16) vorgesehen ist.

2. Einrichtung nach Anspruch 1, dadurch gekennzeichnet, daß die Sensoren (10) als Plattenkondensatoren ausgeführt sind.

3. Einrichtung nach den Ansprüchen 1 oder 2, dadurch gekennzeichnet, daß die Sensoren (10), die Auswerteeinheit (11), das Warnanzeigegerät (12) und die Signalaufbereitungsschaltung (16) ortsfest in das Bremssystem (1) integriert sind, wobei das Warnanzeigegerät (12) am Armaturenbrett zugeordnet ist.

## Claims

1. Device for monitoring a hydraulic fluid, in particular a brake fluid in a braking system of a vehicle for water contained therein, which is located in at least one of the pressure chambers of the braking system, characterised in that a sensor (10) is respectively located in the brake saddle (14) of each brake on the front and rear axle of the vehicle, its front end extending with its measuring surface into the pressure chamber (8) of the brake piston (9) and that the sensors (10) are connected to an electronic evaluation unit (11) with a warning indicator (12) and that a signal-processing circuit (16) is provided close to the sensors (10) between each sensor (10) and the evaluation unit (11).

2. Device according to claim 1, characterised in that the sensors (10) are constructed as plate capacitors.

3. Device according to claims 1 or 2, characterised in that the sensors (10), the evaluation unit (11), the warning indicator (12) and the signal-processing circuit (16) are integrated in a stationary manner in the braking system (1), the warning indicator (12) being associated with the dashboard.

## Revendications

1. Dispositif de surveillance d'un liquide hydraulique, en particulier d'un liquide de frein dans un système de freinage d'un véhicule, pour contrôler l'eau contenue, placé dans l'une au moins des chambres de pression du système de freinage, caractérisé en ce que chaque capteur (10) est placé dans l'étrier (14) de chaque frein respectif de l'essieu avant et de l'essieu arrière du véhicule, en faisant saillie frontalement, par sa surface de mesure, dans la chambre de pression (8) du piston de frein (9) et en ce que les capteurs (10) sont reliés à une unité de traitement électronique (11) comportant un dispositif de signalisation (12), et en ce qu'on prévoit entre chaque capteur (10) et l'unité de traitement (11), près des capteurs (10), un montage de régénération de signaux (16).

2. Dispositif selon la revendication 1, caractérisé en ce que les capteurs (10) sont réalisés sous la forme de condensateurs à lames.

3. Dispositif selon la revendication 1 ou 2, caractérisé en ce que les capteurs (10), l'unité de traitement (11), le dispositif de signalisation (12) et le montage de régénération de signaux (16), sont intégrés fixement dans le système de freinage (1), le dispositif de signalisatior (12) étant rattaché au tableau de bord.

# FIG.1

FIG.2

FIG.3

14

9

15

6

10

8

9

14

9

8

6

9

15a

10